Europäisches Patentamt

(19) European Patent Office

Office européen des brevets

(11) Publication number : **0 408 562 B1**

(12) **EUROPEAN PATENT SPECIFICATION**

(45) Date of publication of patent specification :
19.11.92 Bulletin 92/47

(51) Int. Cl.⁵ : **A61M 25/00, A61L 29/00**

(21) Application number : 89901336.1

(22) Date of filing : 22.12.88

(86) International application number :
PCT/GB88/01141

(87) International publication number :
WO 89/05671 29.06.89 Gazette 89/14

(54) CATHETER.

(30) Priority : 23.12.87 GB 8729977

(43) Date of publication of application :
23.01.91 Bulletin 91/04

(45) Publication of the grant of the patent :
19.11.92 Bulletin 92/47

(84) Designated Contracting States :
AT BE CH DE FR GB IT LI LU NL SE

(56) References cited :
EP-A- 0 207 250
WO-A-86/02561
DE-A- 3 115 763
GB-A- 2 120 947
US-A- 3 598 127
US-A- 3 695 921
US-A- 4 054 139

(73) Proprietor : THE VICTORIA UNIVERSITY OF
MANCHESTER
Oxford Road
Manchester M13 9PT (GB)

(72) Inventor : SUTTON, Terence, Michael
18 Pampas Close Highwoods
Colchester Essex C04 4ST (GB)
Inventor : HUKINS, David, William, Laurence
39 South Drive Chorltonville
Manchester M21 2DZ (GB)
Inventor : COX, Averil, Jane
17 Alderbank Horwich Bolton
Lancashire BL6 7RF (GB)
Inventor : CAPSTICK, Ian
20 Marine Court Marine Parade West
Clacton on Sea Essez CO15 1ND (GB)
Inventor : BIBBY, Joanna, Margaret
2 Hollock Lea Cragg Road Hebdon Bridge
Yorkshire HX7 5RT (GB)

(74) Representative : Ajello, Michael John
207 Moss Lane
Bramhall, Stockport, Cheshire SK7 1BA (GB)

Jouve, 18, rue Saint-Denis, 75001 PARIS

## Description

This invention relates to catheters, in particular to medical catheters such as those employed in draining or feeding fluids from or to a patient. Among the most common examples are those used in the lower or upper urinary tract or in the cardiovascular system.

In order to perform their required duty medical catheters must of necessity come into contact with the body organs and remain in place for a period of time which may be prolonged. Many problems however arise from the use of indwelling catheters. For example in the case of urinary drainage, a build-up can occur on the catheter of infecting bacteria or of crystalline deposits, this latter condition being known as encrustation or concretion. If the build-up leads to blockage of the catheter then the drainage of urine ceases and the catheter has to be prematurely removed, often with considerable discomfort to the patient and sometimes with associated damage to the lining of the organ through which the catheter passes and further resultant problems such as strictures and urethritis.

In the case of catheters used to introduce fluids into a patient, for example by intravenous feeding, it is important that the catheter is not also a source of introduction of infection.

Attempts have been made to prevent catheter-associated infection by known anti-bacterial treatments, but these tend not to be fully effective in removing infecting bacteria, for example those which secrete the enzymes known collectively as ureases. Such enzymes are a major factor in causing encrustation of urinary catheters. The introduction of an acidic solution to wash out the bladder can be effective in removing deposits but does not prevent a recurrence of the problem which created them.

Other proposals have included soaking or coating the catheter with such materials as silver salts, metallic silver and general antiseptic solutions. For example US patent 4 054 139 describes and claims a catheter tube with a silver-bearing material on its exterior and interior surfaces.

Modifications have been proposed to catheters for other purposes, for example to assist insertion of a suprapubic catheter with minimum discomfort to the patient. Thus GB patent 2 120 947 describes and claims a catheter having a shaped fairing to facilitate entry of the catheter through tissue, the fairing being separable from the catheter upon withdrawal of the entry instrument and being of a urine-dispersible material such that it becomes flushed from the bladder during subsequent drainage.

The present invention is concerned with modifications to the material of the catheter so as to prolong the period over which the catheter can be left in place in the body before the onset of the indwelling problems described above.

In its broadest aspect, the invention provides a catheter for drainage or collection of a bodily fluid which catheter comprises a portion which throughout its period of use is retained within a body cavity, characterised in that at least part of the said portion supports within the tube a solid biodegradable material, such that a controlled and continuous release of biodegradation products takes place throughout the said period.

The term "biodegradable" as used herein refers to a material which will degrade in the biological fluids to which it will be subjected. The choice can be made for example from such materials as polyglycolides, polylactides, polybutyrates, collagen, gelatin and hydrogels, the specific choice of biodegradable material and the manner in which it is incorporated into the catheter structure being dictated by the intended use of the catheter.

The essential element of a catheter is an elongated tube to provide a fluid-carrying lumen. Catheters according to the invention employ a composite structure including a tube of conventional (non-biodegradable) flexible material supporting the biodegradable material. Suitable materials for the supporting tube include natural rubber latex, polyvinyl chloride, polyurethane, polytetrafluorethylene and silicone rubbers.

The usual form of catheter tube has two or more holes through the tube wall close to the distal end (i.e. within the body) to permit passage of a bodily fluid into the tube. At its proximal end (i.e. external to the body) it is usually provided with connection means for attachment to a drainage tube or reservoir. In general the connection means should not include any biodegradable material since biodegradation at this point could lead to leakage or to detachment of the catheter tube from the associated tube or reservoir.

The biodegradable material must be either integral with any other material used in the catheter tube or be firmly affixed to the catheter tube so that at least part of the biodegradable material remains attached to the catheter tube throughout substantially all of the period in which the catheter tube is retained in the body cavity.

The use of a biodegradable material according to the invention provides for an increase in the length of time for which a catheter can be retained without discomfort to the patient. By use in a variety of structures as described in greater detail below the biodegradation reduces the problems that otherwise occur with indwelling catheters, especially in terms of resisting infection, encrustation and blockage.

The biodegradable material can for example be a lining within the tube, a patch or patches on the inner surface of the tube, a stripe or stripes running along the inner surface of the tube, a plug or plugs in the tube, or any combination of these different configurations. For example, with the objective of preventing barriers to fluid flow, the biodegradable material can be applied as a multiplicity of layers within the tube such that as the deposits build up, the uppermost layer of the biodegradable material is shed in turn, taking the deposited material away with it and leaving a new outer layer of biodegradable material and a cleared passageway within the catheter.

The biodegradable material can be applied to the tube in combination with one or more additives which effect a medicating treatment within the body cavity in which the catheter is located. A variety of additives can be chosen with a view to achieving such benefits as inhibiting enzymes secreted by infecting bacteria, inhibiting the deposit of extraneous matter, dissolving deposited material, and achieving general anti-microbial, antiseptic or antibiotic action. For example acidic materials can be employed to acidify urine or accelerate the acidification process. Inhibitors can be employed to inhibit or denature enzymes such as urease or to inhibit mineral deposition. Anti-microbial or antibiotic materials can be used to reduce infection and to target treatment in selected areas. Typical additives for use in such ways include citric acid, tartaric acid, citrates, acetohydroxamic acid, chlorhexidine and silver compounds.

In one particularly advantageous embodiment of the invention, the biodegradable material is chosen from a substance which itself breaks down to produce the beneficial medical treatment. Thus for urine-removal catheters, biodegradable polymers can be utilised which hydrolyse in the urinary fluids to give acidic degradation products. Examples of such polymers are polylactides, polyglycolides and polybutyrates. The preferred polymers according to the invention for use in this application are poly-L-lactide, poly-L-glycolide and poly-3-hydroxybutyric acid. Such polymers give a continuous release of acidic materials into the urinary tract throughout the period in which the catheter is retained in place and thus provide an anti-encrustation regime at all times.

In further preferred versions of the invention the medicating additive is either absorbed in or encapsulated by the biodegradable material so as to give a controlled and continuous release of the additive as the biodegradation proceeds. Encapsulation can be achieved by several different forms of catheter having a composite construction. Thus, in one embodiment a layer of porous material, for example of natural rubber latex foam, is first applied to the catheter tube, the additive is impregnated in the porous layer, for example by dipping the latex-coated tube into the material, and a layer of biodegradable material is applied as a coating over the porous layer. In a variation of this embodiment, the biodegradable material, for example in the form of solid granules, can itself be impregnated in the porous material rather than being present as a coating.

In such composite structures it is important to ensure that the biodegradable material is firmly secured either by mechanical means or adhesives, to the catheter tube, so that biodegradable material remains attached to the catheter tube throughout at least the greater part of the indwelling period. If desired when adhesive bonding is employed, the composite structure can include a matrix comprising a bonding aid, for example of fibrous cotton or paper, which displays a strong bonding to the chosen adhesive.

In a particularly convenient form of composite construction the biodegradable material is present as a plug located within the catheter tube at or immediately adjacent to the distal end and securely attached to the tube, for example by adhesive, by complementary shapes of the plug and tube or by a retaining membrane. Several different configurations of plug satisfy the requirements of the invention. Thus it can be for example

(a) a single solid piece of biodegradable material,
(b) a shell of biodegradable material directly encapsulating a medicating additive, or
(c) a shell of biodegradable material enclosing a solid material impregnated with a medicating additive.

One advantageous configuration of tubular structure to ensure retention of the plug is an inner annulus within the tube adjacent to the plug.

The biodegradable material should be chosen according to the intended use of the catheter and in particular to the intended length of time for which the catheter is to be left in place in the body cavity. A range of rates of biodegradation, and thus control over the rate and period over which a beneficial material is released, is provided by choice of biodegradable material according to the rate at which it hydrolyses in the bodily fluids. For example in urine polybutyrates hydrolyse more slowly than polyglycolides which in turn hydrolyse more slowly than polylactides. It is also possible to reduce the biodegradability of such polymers by appropriate cross linking reactions during their production. Thus the invention conveniently provides for a catheter giving a controlled and continuous release of beneficial material over a prolonged period, for example of 14 to 21 days or even longer.

The preferred quantity of beneficial material to be released from the catheter varies according to the material in question, to the form of encapsulation and to the required rate of release. In general the quantity is within the range 10 to 1000 mg, the lower limit being dictated by the need to include sufficient material to be effective, the upper limit by the dimensions of the catheter. When present as a lining on the catheter tube the biodegradable material has a typical thickness in the range 20 to 40 µm and preferably extends over the whole of the portion of the catheter tube which is to be located in the body cavity.

The invention offers the advantage that the catheter can be safely left in place in its desired application for the indwelling period without fear of the onset of infection or build-up of blockages and thereby avoids the need for premature and painful removal in the event of troublesome complications.

The controlled and continuous release of biodegradation products from a catheter according to the invention, throughout the indwelling period, makes it possible to prolong the indwelling period without discomfort to the patient. Several preferred versions of the catheter according to the invention additionally give a controlled and continuous release of medical treatment materials throughout the indwelling period.

The invention is described below with reference to the accompanying figures, in which

Figure 1 is a general view of a tubular catheter of the type known as a Foley catheter and incorporating biodegradable material according to the invention so as to improve its properties for use as an urethral urine drain.

Figure 2 is a sectional view of the tip of the catheter shown in Figure 1.

Figure 3 shows in cross section a portion of a catheter of generally similar type to that shown in Figure 1 and also for use in urethal urine drainage but with a different configuration of biodegradable material from that shown in Figure 2. The portion illustrated in Figure 3 is taken from a position equivalent to that indicated by lines A-A′ in Figure 1. In order to emphasize the cross sectional construction the dimensions in Figure 3 are not strictly to scale.

Figure 4 is an enlarged cross sectional view, taken along a line equivalent to B-B′ of Figure 1 (but not showing the retention balloon 14), of a further type of internal construction for a catheter similar to that shown in Figure 1.

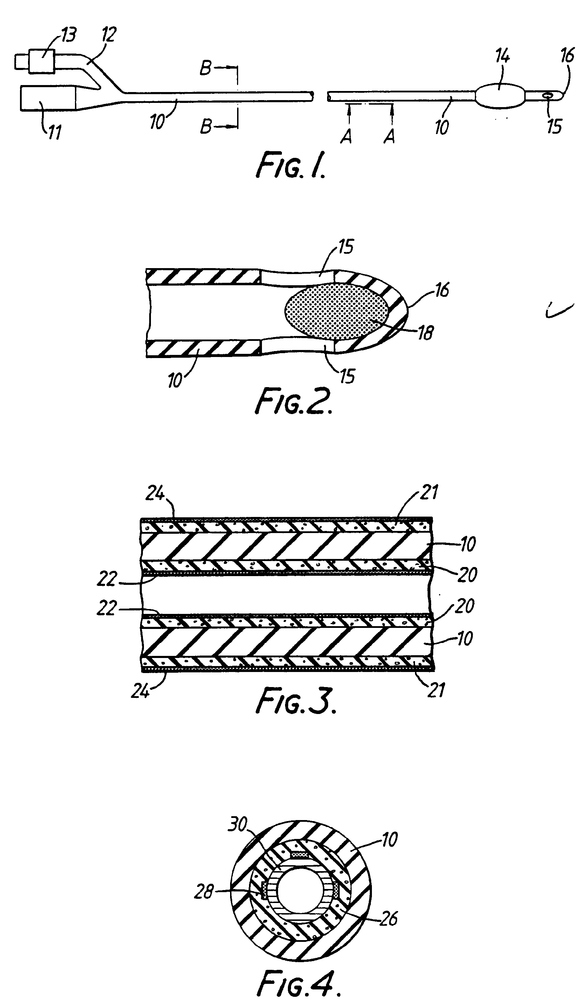

The catheter shown in Figures 1 and 2 comprises a tube 10 of natural rubber latex having a connection funnel 11 for attachment to a drainage tube (not shown) and an inflation funnel 12, with associated valve 13, for use in the inflation of a retention balloon 14. The tube 10 has two oval-shaped eye holes 15 adjacent to its distal end 16 so as to permit urine drainage into the tube 10. The distal end 16 has a gently curved surface to facilitate insertion into the body cavity. A biodegradable plug 18 (not visible in Figure 1) of ovoid shape and formed of poly-L-glycolide is located within the tube 10 at its distal end 16 and is secured furmly in place by a combination of the complementary shapes of its outer surface and the tube's inner surface and of an acrylic adhesive on the said surfaces. The plug 18 protrudes slightly into the urine channel provided by the eyeholes 15 and tube 10. The protrusion ensures good contact between the plug 18 and the urine flowing through the said channel. The catheter tube 10 is a 16 Charrière unit with an internal diameter of 2.7 mm and the eyeholes have an axial length of 6.7 mm. The biodegradable plug 18 has a volume of 15 mm³.

Once the catheter has been inserted into the urethra and urine enters the drainage lumen, the urine begins the hydrolysis of the polyglycolide, thereby releasing glycolic acid into the urethra. The release continues throughout the period of retention of the catheter, which in this particular example is typically about 14 days. It is desirable that the catheter be withdrawn as soon as the release of glycolic acid is complete.

The catheter having a portion as shown in Figure 3 is generally similar to that shown in Figure 1, i.e. it has a tube 10 of natural rubber latex, a connection funnel 11, an inflation funnel 12 and valve 13, and two oval-shaped eye-holes 15 adjacent its distal end 16. Unlike the version illustrated in Figure 2 it does not include a biodegradable plug. Instead the tube 10 in this instance has an inner and outer coating, 20 and 21 respectively, of latex foam and biodegradable encapsulating layers, respectively 22 and 24, of poly-3-hydroxybutyric acid on the latex foam coatings 20 and 21. Before application of the encapsulating layers 22 and 24, the tube 10 is dipped into chlorhexidine which is thereby impregnated in the foam coatings 20 and 21. On a size 16 Charrière catheter as illustrated the coatings 20 and 21 each have a thickness of 100 μm and the biodegradable layers 22 and 24 have an initial thickness of 25 μm.

When placed in the urethra a catheter of the type shown in Figure 3 first undergoes hydrolysis of the biodegradable layers 22 and 24, which releases hydroxybutyric acid into the urine. As the hydrolysis proceeds however the layers 22 and 24 gradually reduce in thickness and allow the chlorhexidine impregnated in the foam coatings 20 and 21 to permeate through them and to effect an anti-microbial action in addition to the acidic treatment effected by the hydroxybutyric acid.

In the version of catheter illustrated in Figure 4, the tube 10 is again externally similar to those described in Figures 1 to 3 but internally has a latex foam layer 26, three longitudinal stripes 28 of poly-L-glycolide and a lining 30 of hydrogel. The latex foam layer 26 is impregnated with an antibiotic material.

As the hydrogel biodegrades this version of catheter releases both antibiotic material from the foam layer 26 and acidic degradation products of the poly-L-glycolide stripes, thereby giving simultaneous treatment to inhibit infection and increase acidity.

## Claims

1. A catheter for drainage or collection of a bodily fluid, which catheter comprises a tube of non-biodegradable flexible material having a portion which throughout its period of use is retained within a body cavity, characterised in that at least part of the said portion supports within the tube a solid biodegradable material, such that a controlled and continuous release of biodegradation products takes place throughout the said period.

2. A catheter as claimed in claim 1, characterised in that the biodegradable material is in the form of a multiplicity of layers within the tube.

3. A catheter as claimed in claim 1 or claim 2, characterised in that the tube also supports one or more additives which effect a medicating treatment within the body cavity.

4. A catheter as claimed in claim 3, characterised in that the or each additive is selected from citric acid, tartaric acid, citrates, acetohydroxamic acid, chlorhexidine and silver compounds.

5. A catheter as claimed in claim 3 or claim 4, characterised in that the or each additive is absorbed in the biodegradable material.

6. A catheter as claimed in claim 3 or claim 4, characterised in that the biodegradable material encapsulates the or each additive.

7. A catheter as claimed in claim 3 or claim 4, characterised in that the or each additive is impregnated in the material of the catheter.

8. A catheter as claimed in claim 7, characterised in that the tube supports a layer of porous material and the or each additive is impregnated in the porous layer.

9. A catheter as claimed in claim 3 or claim 4, characterised in that the tube supports a layer of porous material and the biodegradable material is impregnated in the porous layer.

10. A catheter as claimed in any one of claims 1 to 8, characterised in that the biodegradable material is a lining within the tube.

11. A catheter as claimed in any one of claims 1 to 8, characterised in that the biodegradable material is a patch or patches on the inner surface of the tube.

12. A catheter as claimed in any one of claims 1 to 8, characterised in that the biodegradable material is a stripe or stripes running along the inner surface of the tube.

13. A catheter as claimed in any one of claims 1 to 8, characterised in that the biodegradable material is in the form of a plug or plugs in the tube.

14. A catheter as claimed in claim 13, characterised in that the biodegradable material is in the form of a plug located within the catheter tube at or immediately adjacent to the distal end thereof and securely attached thereto.

15. A catheter as claimed in claim 14, characterised in that the plug is a solid piece of biodegradable material.

16. A catheter as claimed in claim 14, characterised in that the plug is a shell of biodegradable material directly encapsulating a medicating additive.

17. A catheter as claimed in claim 14, characterised in that the plug is a shell of biodegradable material enclosing a solid material impregnated with a medicating additive.

18. A catheter as claimed in any one of claims 14 to 17, characterised in that the tube has an inner annulus adjacent to the plug.

19. A catheter as claimed in any preceding claim, characterised in that the biodegradable material is secured by adhesive means.

20. A catheter as claimed in claim 19, characterised in that a bonding aid is employed in association with the adhesive.

21. A catheter as claimed in any preceding claim, characterised in that the biodegradable material is of a type which degrades within the body cavity to effect a medicating treatment.

22. A catheter as claimed in claim 21, characterised in that the biodegradable material is selected from polylactides, polyglycolides and polybutyrates.

**Patentansprüche**

1. Katheter zum Ableiten oder Sammeln einer Körperflüssigkeit, der eine Röhre aus flexiblem, biologisch nichtaubbaubarem Material aufweist, die einen Teil hat, der während seiner gesamten Gebrauchsperiode innerhalb eines Körperhohlraums gehalten wird, dadurch gekennzeichnet, daß wenigstens ein Teil des genannten Teils innerhalb der Röhre einen Feststoff aus biologisch abbaubarem Material trägt, so daß ein kontrolliertes und fortgesetztes Freisetzen von Produkten des biologischen Abbaus während der gesamten Zeitspanne erfolgt.

2. Katheter nach Anspruch 1, dadurch gekennzeichnet, daß das biologisch abbaubare Material in Form von mehreren Schichten innerhalb der Röhre vorliegt.

3. Katheter nach Anspruch 1 oder Anspruch 2, dadurch gekennzeichnet, daß die Röhre außerdem einen oder mehrere Zusätze trägt, die eine Medikamentenbehandlung innerhalb des Körperhohlraums bewirken.

4. Katheter nach Anspruch 3, dadurch gekennzeichnet, daß der oder jeder Zusatz unter Zitronensäure, Weinsäure, Citraten, Acethydroxamsäure, Chlohexidin und Silberverbindungen ausgewählt ist.

5. Katheter nach Anspruch 3 oder Anspruch 4, dadurch gekennzeichnet, daß der oder jeder Zusatz in dem biologisch abbaubaren Material absorbiert ist.

6. Katheter nach Anspruch 3 oder Anspruch 4, dadurch gekennzeichnet, daß das biologisch abbaubare Material den oder jeden Zusatz einkapselt.

7. Katheter nach Anspruch 3 oder Anspruch 4, dadurch gekennzeichnet, daß das Material des Katheters mit dem oder jeden Zusatz getränkt ist.

8. Katheter nach Anspruch 7, dadurch gekennzeichnet, daß die Röhre eine Schicht porösen Materials trägt und daß die poröse Schicht mit dem oder jedem Zusatz getränkt ist.

9. Katheter nach Anspruch 3 oder Anspruch 4, dadurch gekennzeichnet, daß die Röhre eine Schicht porösen Materials trägt und daß die poröse Schicht mit dem biologisch abbaubaren Material getränkt ist.

10. Katheter nach einem der Ansprüche 1 bis 8, dadurch gekennzeichnet, daß das biologisch abbaubare Material eine Auskleidung innerhalb der Röhre ist.

11. Katheter nach einem der Ansprüche 1 bis 8, dadurch gekennzeichnet, daß das biologisch abbaubare Material aus einem oder mehreren Flecken auf der inneren Oberfläche der Röhre besteht.

12. Katheter nach einem der Ansprüche 1 bis 8, dadurch gekennzeichnet, daß das biologisch abbaubare Material aus einem oder mehreren Streifen besteht, die längs der inneren Oberfläche der Röhre verlaufen.

13. Katheter nach einem der Ansprüche 1 bis 8, dadurch gekennzeichnet, daß das biologisch abbaubare Material in Form von einem oder mehreren Stopfen in der Röhre vorliegt.

14. Katheter nach Anspruch 13, dadurch gekennzeichnet, daß das biologisch abbaubare Material in Form eines Stopfens vorliegt, der in der Katheterröhre an oder unmittelbar benachbart zu dem distalen Ende derselben angeordnet und fest daran angebracht ist.

15. Katheter nach Anspruch 14, dadurch gekennzeichnet, daß der Stopfen ein massives Stück biologisch abbaubaren Materials ist.

16. Katheter nach Anspruch 14, dadurch gekennzeichnet, daß der Stopfen eine Mülle aus biologisch abbaubarem Material ist, die einen Medikamentenzusatz direkt einkapselt.

17. Katheter nach Anspruch 14, dadurch gekennzeichnet, daß der Stopfen eine Hülle aus biologisch abbaubarem Material ist, die ein massives Material umschließt, das mit einem Medikamentenzusatz getränkt ist.

18. Katheter nach einem der Ansprüche 14 bis 17, dadurch gekennzeichnet, daß die Röhre einen inneren Kreisring an dem Stopfen hat.

19. Katheter nach irgendeinem vorhergehenden Anspruch, dadurch gekennzeichnet, daß das biologisch abbaubare Material mittels Klebstoff befestigt ist.

20. Katheter nach Anspruch 19, dadurch gekennzeichnet, daß eine Verbindungshilfe zusammen mit dem Klebstoff benutzt wird.

21. Katheter nach irgendeinem vorhergehenden Anspruch, dadurch gekennzeichnet, daß das biologisch abbaubare Material von einem Typ ist, der innerhalb des Körperhohlraums abgebaut wird, um eine Medikamentenbehandlung zu bewirten.

22. Katheter nach Anspruch 21, dadurch gekennzeichnet, daß das biologisch abbaubare Material unter Polylactiden, Polyglycoliden und Polybutyraten ausgewählt ist.

## Revendications

1. Un cathéter pour le drainage ou la collecte d'un fluide du corps, ledit cathéter comprenant un tube en matériau souple, non-biodégradable dont une partie, au cours de sa période d'utilisation, est logée dans une cavité du corps humain, caractérisé en ce qu'une portion au moins de ladite partie supporte à l'intérieur du tube un matière biodégradable solide, de manière à assurer une libération contrôlée et continue des produits de biodégradation pendant toute ladite période.

2. Un cathéter selon la revendication 1, caractérisé en ce que le matière biodégradable est sous forme d'une pluralité de couches à l'intérieur du tube.

3. Un cathéter selon la revendication 1 ou 2, caractérisé en ce que le tube supporte également un ou plusieurs additifs assurant un traitement médical dans la cavité du corps.

4. Un cathéter selon la revendication 3, caractérisé en ce que l'additif ou que chaque additif est choisi parmi l'acide citrique, l'acide tartrique, les citrates, l'acide acétohydroxamique, la chlorhexidine et des composés d'argent.

5. Un cathéter selon la revendication 3 ou 4, caractérisé en ce que l'additif ou que chaque additif est absorbé par la matière biodégradable.

6. Un cathéter selon la revendication 3 ou 4, caractérisé en ce que l'additif ou que chaque additif est enrobé dans la matière biodégradable.

7. Un cathéter selon la revendication 3 ou 4, caractérisé en ce que l'additif ou que chaque additif est imprégné dans le matériau du cathéter.

8. Un cathéter selon la revendication 7, caractérisé en ce que le tube supporte une couche de matériau poreux et que la couche poreuse est imprégnée de l'additif ou de chaque additif.

9. Un cathéter selon la revendication 3 ou 4, caractérisé en ce que le tube supporte une couche de matériau poreux et que la couche poreuse est imprégnée de matière biodégradable.

10. Un cathéter selon l'une quelconque des revendications 1 à 8, caractérisé en ce que la matière biodégradable est constituée par un revêtement à l'intérieur du tube.

11. Un cathéter selon l'une quelconque des revendications 1 à 8, caractérisé en ce que la matière biodégradable est constituée par un dépôt en forme d'une ou de plusieurs plaques sur la surface intérieure du tube.

12. Un cathéter selon l'une quelconque des revendications 1 à 8, caractérisé en ce que la matière biodégradable est constituée par une ou plusieurs bandes s'étendant le long de la surface intérieure du tube.

13. Un cathéter selon l'une quelconque des revendications 1 à 8, caractérisé en ce que la matière biodégradable est réalisée sous forme d'un ou de plusieurs tampons ou pastilles dans le tube.

14. Un cathéter selon la revendication 13, caractérisé en ce que la matière biodégradable est réalisée en forme de tampon ou de pastille, placé à l'intérieur du tube de cathéter, à son extrémité distale ou à proximité immédiate, et fixé solidement sur celle-ci.

15. Un cathéter selon la revendication 14, caractérisé en ce que le tampon ou pastille est une fragment massif de matière biodégradable.

16. Un cathéter selon la revendication 14, caractérisé en ce que le tampon ou pastille est constitué par une enveloppe de matière biodégradable enfermant directement un additif médicamenteux.

17. Un cathéter selon la revendication 14, caractérisé en ce que le tampon ou pastille est constitué par une enveloppe de matière biodégradable enfermant directement une matière solide imprégnée d'un additif médicamenteux.

18. Un cathéter selon l'une quelconque des revendications 14 à 17, caractérisé en ce que le tube comprend un anneau intérieur adjacent au tampon.

19. Un cathéter selon l'une quelconque des précédentes revendications, caractérisé en ce que la matière biodégradable est fixée par des moyens en forme d'adhésif.

20. Un cathéter selon la revendication 19, caractérisé en ce qu'un agent auxiliaire de collage est utilisé en association avec l'adhésif.

21. Un cathéter selon l'une quelconque des précédentes revendications, caractérisé en ce que la matière biodégradable est d'un type se dégradant à l'intérieur de la cavité du corps humain, de manière à effectuer un traitement médical.

22. Un cathéter selon la revendication 21, caractérisé en ce que la matière biodégradable est choisie parmi les polylactides, les polyglycolides et les polybutyrates.

FIG. I.

FIG. 2.

FIG. 3.

FIG. 4.